# EUROPEAN PATENT SPECIFICATION

(11) **EP 4 380 434 B1**
(45) Date of publication and mention of the grant of the patent: **29.10.2025**
(21) Application number: 22768255.6
(22) Date of filing: 03.08.2022
(51) Int. Cl.: A61B 5/00, A61B 5/05, A61B 5/24, A61N 2/00, A61N 2/02

(54) **METHOD FOR LOCALISING ACTIVATION UNDER TRANSCRANIAL MAGNETIC STIMULATION**
VERFAHREN ZUR LOKALISIERUNG DER AKTIVIERUNG UNTER TRANSKRANIELLER MAGNETSTIMULATION
PROCÉDÉ DE LOCALISATION D'ACTIVATION SOUS STIMULATION MAGNÉTIQUE TRANSCRÂNIENNE

(30) Priority: 03.08.2021 FI 20217127
(43) Date of publication of application: 12.06.2024
(73) Proprietor: Aalto University Foundation sr, 00076 Aalto (FI)
(72) Inventor: KATAJA, Juhani, 02150 Espoo (FI); LAAKSO, Ilkka, 02150 Espoo (FI); MATILAINEN, Noora, 02150 Espoo (FI); SOLDATI, Marco, 02150 Espoo (FI)
(74) Representative: Papula Oy
(86) International application number: PCT/EP2022/071849
(87) International publication number: WO 2023/012228

(56) References cited:
- LEFAUCHEUR ET AL: "Why image-guided navigation becomes essential in the practice of transcranial magnetic stimulation", NEUROPHYSIOLOGIE CLINIQUE - CLINICAL NEUROPHYSIOLOGY, ELSEVIER, PARIS, FR, vol. 40, no. 1, 1 March 2010 (2010-03-01), pages 1 - 5, XP026964478, ISSN: 0987-7053, [retrieved on 20091124]
- JIANG YIHAN ET AL: "Targeting brain functions from the scalp: Transcranial brain atlas based on large-scale fMRI data synthesis", NEUROIMAGE, ELSEVIER, AMSTERDAM, NL, vol. 210, 22 January 2020 (2020-01-22), XP086071059, ISSN: 1053-8119, [retrieved on 20200122], DOI: 10.1016/J.NEUROIMAGE.2020.116550
- TERVO AINO E ET AL: "Automated search of stimulation targets with closed-loop transcranial magnetic stimulation", NEUROIMAGE, ELSEVIER, AMSTERDAM, NL, vol. 220, 25 June 2020 (2020-06-25), XP086288644, ISSN: 1053-8119, [retrieved on 20200625], DOI: 10.1016/J.NEUROIMAGE.2020.117082
- SHALINI NARAYANA ET AL: "Electrophysiological and functional connectivity of the human supplementary motor area", NEUROIMAGE, ELSEVIER, AMSTERDAM, NL, vol. 62, no. 1, 21 April 2012 (2012-04-21), pages 250 - 265, XP028494485, ISSN: 1053-8119, [retrieved on 20120505], DOI: 10.1016/J.NEUROIMAGE.2012.04.060
- KING M ET AL: "The handyman's brain: A neuroimaging meta-analysis describing the similarities and differences between grip type and pattern in humans", NEUROIMAGE, vol. 102, 15 November 2014 (2014-11-15), pages 923 - 937, XP029078771, ISSN: 1053-8119, DOI: 10.1016/J.NEUROIMAGE.2014.05.064

## Description

### FIELD

The present disclosure relates to a method for localising activation under transcranial magnetic stimulation (TMS). In particular, the disclosure relates to a method for localising cortical activation sites under transcranial magnetic stimulation.

### BACKGROUND

By correlating an ensemble of electromagnetic stimuli and their corresponding response signals the location in the brain that is being activated can be determined. At simplest, the activating stimulator coil can be moved across the area of interest over the scalp and the activation location can be determined to be that location on the cortex where the stimulating electric field of the coil location that elicits strongest response signal is at its maximum value (Ru-ohonen & Karhu, 2010). More involved methods take in to account the conductivity of the tissues of the head (Bungert, et al., 2016; Weise, et al., 2020).

Additionally, Yihan Jiang et al (NeuroImage 210, 2020, 116550) discloses a transcranial brain atlas based on large-scale fMRI data synthesis, Tervo Aino E et al (NeuroImage 220, 2020, 117082) discloses automated search of stimulation targets with closed-loop transcranial magnetic stimulation, Shalini Nara-yana et al (NeuroImage 62, 2012, 250-265) discloses electrophysiological and functional connectivity of the human supplementary motor area and M. King et al (NeuroImage 102, 2014, 923-937) discloses a neuroimaging meta-analysis describing the similarities and differences between grip type and pattern in humans.

The main drawbacks of the existing approaches in finding the activation location are as follows:
1. A single location is assumed.
2. Ad-hoc measures of correlation are used.
3. The directionality information of the electric field is not fully utilized.
4. A measure of goodness for the localisation is not available.

### OBJECTIVE

An objective is to provide an improved method for TMS. In particular, it is an objective to alleviate the disadvantages mentioned above.

### SUMMARY

In accordance with the present disclosure, it has been found that finding the activation location under TMS can be markedly improved utilizing a probabilistic method. In particular, it has been found that by computing activation parameter statistics with activation location as an activation parameter, where the activation parameter statistics further comprises a probability distribution of the activation location, results in an improved method for TMS. This is particularly evident in comparison to ad hoc measures of the prior art.

According to a first aspect, a method, executed using computing means, for localising activation under TMS is provided. The method may be utilized, in particular, for localising cortical activation sites under TMS. The activation sites may alternatively be referred herein as activation locations. The method comprises computing activation parameter statistics. For this purpose, activation location is used as an activation parameter. Importantly, the activation parameter statistics computed comprise a probability distribution of the activation location. This allows a marked improvement over the existing ad hoc methods.

In an embodiment, the activation parameter statistics are computed with preferred direction of activation as an additional activation parameter.

In an embodiment, the activation parameter statistics are computed with activating electric field threshold (EFT) as an additional activation parameter.

In an embodiment, the activation parameter statistics comprises a conditional expectation of the preferred direction of activation divided by the activating EFT at each activation location.

In an embodiment, the activation parameter statistics comprises a conditional expectation of the activating EFT at each activation location.

In an embodiment, the activation parameter statistics comprises the preferred direction of activation at each activation location.

In an embodiment, the activation parameter statistics comprises a likelihood measure that response signals originate from a unique "activation location - preferred direction of activation - activating EFT" -triple.

In an embodiment, the activation parameter statistics are computed with prior information including a minimal value of electric field modulus at which activation may occur.

In an embodiment, the activation parameter statistics are computed with prior information including accuracy information about response signals.

The method may thus leverage prior information, in particular of minimum EFT and/or uncertainty estimate of the response signal threshold values.

In an embodiment, the activation is localised within one or more threshold probability volumes for which an integral of the probability distribution exceeds a threshold probability. The volumes, as individual volumes, may be unbroken, and even simply connected, but two or more of the one or more threshold probability volumes may be separate. Consequently, multiple separate activation sites may be concurrently localised.

In an embodiment, the threshold probability is 90 percent or more, in particular 95 percent or more.

The method may involve using any or all of the activation parameters described above. It may also involve computing the activation parameter statistics with any or all of the activation parameter statistics as described above.

The activation parameter statistics may be computed based on TMS and response signal threshold measurement. The method may thus involve correlating an ensemble of electromagnetic stimuli and their corresponding response signals to determine the location in the brain that is being activated (by the TMS). However, the method allows determining multiple activation locations that may be concurrent. For example, two or more locations in the cortex of the subject may be responsible for same threshold and/or similar signals. The solutions, in such a case, allows localising the activation to the two or more locations.

According to a second aspect, a computer program product comprises instructions which, when the computer program product is executed by a computer, cause the computer to carry out the method of the first aspect and/or any of its embodiments alone or in combination.

The disclosed solutions may be used in (functional) cortical and/or brain mapping, in particular with TMS. In particular, the solutions can be used in scientific mapping of the human brain. Importantly, any mapping performed in accordance with the solutions may be real-time mapping. In such a use case, the solutions can be used to characterize and/or locate sites in the brain that are associated with certain activities. For example, the solutions can be used in correlating muscular activity to brain activity. The solutions can be also used in navigating for individualized transcranial magnetic stimulation treatment for more targeted stimulation. The solutions can be also used in pre-surgical mapping of a patient's brain. For example, so that a surgeon is given information which tissue may not be operated on to avoid damaging critical locations in the brain. The solutions can be also used to measure brain tissue excitability. This may have utility in monitoring the patient's EFT value, for example after a stroke. In general, the solutions are advantageous in multi-site stimulation, which may not necessarily be related to single sites/activation location but to an underlying network, which may be represented by multiple concurrent activation locations.

In general, the solutions, including the method, may involve, given a measured response signals of known electromagnetic stimuli to human body and electromagnetic anatomical model (EAM) of the excited region, producing statistics of the activation parameters of the excited tissue. The activation parameters may comprise or consist of any or all of
1. the activation location,
2. the preferred direction of activation, and
3. the activating electric field threshold (EFT).

The statistics of the parameters may comprise or consist of any or all of
- a marginal probability distribution of the activation location,
- a conditional expectation of the preferred direction of activation divided by the activating EFT at each location,
- a conditional expectation of activating EFT at each location,
- a conditional expectation of preferred direction at each location, and
- a likelihood measure that the response signals originate from a unique location-direction-EFT triple.
This may involve mappings of the statistics, for example bijective mappings. For example, any or all of the examples given below, such as the marginal probability distribution, may be scaled by one or more constants for the statistics.

The statistics may be outputted in the coordinate frame of the EAM. Prior information about the parameters may also be inputted to the invention. The prior information may include minimal value of electric field modulus at which activation may occur, and restricted region in the excited region where the activation may occur. The prior information may also include accuracy information about the response signals.

Any or all of the solutions disclosed herein may be provided as a piece of computer software, or as a computer program product.

It is to be understood that the aspects and embodiments described above may be used in any combination with each other. Several of the aspects and embodiments may be combined together to form a further embodiment of the invention.

### BRIEF DESCRIPTION OF THE DRAWINGS

The accompanying drawings, which are included to provide a further understanding and constitute a part of this specification, illustrate examples and together with the description help to explain the principles of the disclosure. In the drawings:
**Fig. 1** illustrates an example of usage environment, input data, output data and steps involved in the processing for the solutions,
**Fig. 2** illustrates an example of detailed steps involved in the METHOD part shown in Figure 1, and
**Fig. 3** illustrates a method according to an example.

Like references are used to designate equivalent or at least functionally equivalent parts in the accompanying drawings.

### DETAILED DESCRIPTION

The detailed description provided below in connection with the appended drawings is intended as a description of examples and is not intended to represent the only forms in which the example may be constructed or utilized. However, the same or equivalent functions and structures may be accomplished by different examples.

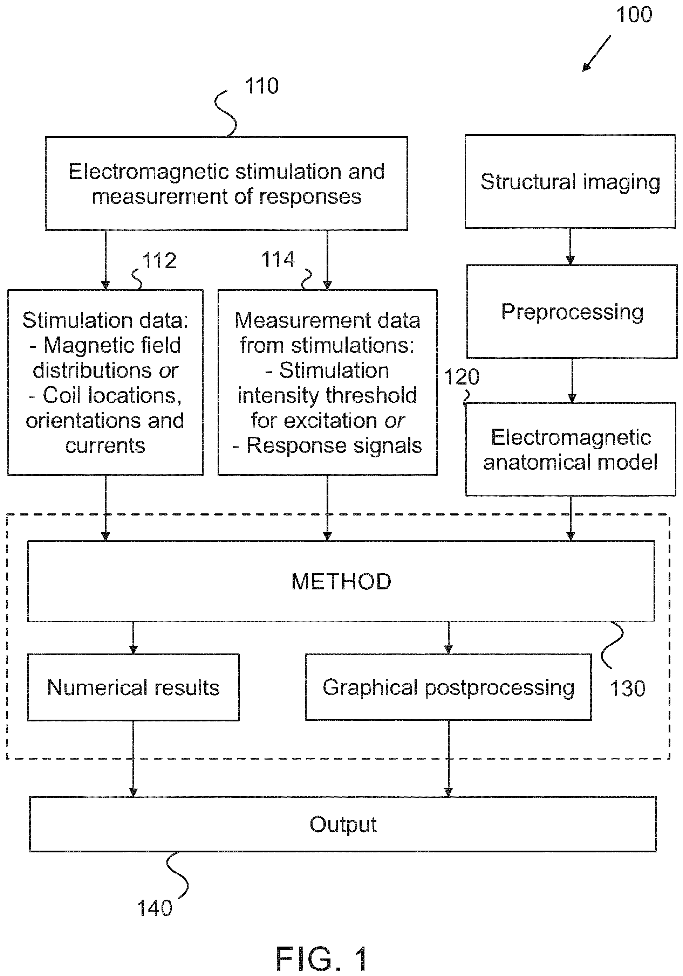

Figure 1 shows an example 100 of usage environment, input data, output data and steps involved in the processing for the solutions. First, the solutions may involve electromagnetic stimulation 110 of a subject, in particular a human subject. This may be carried out by TMS. Upon the stimulation, responses to the stimulation may be measured to provide response signals. This may be referred herein as "response measurement(s)". Correspondingly, stimulation data 112 and/or measurement data 114 may be provided. Stimulation data corresponds to electromagnetic stimulation performed and it may include magnetic field distributions and/or coil locations, orientations and currents. The number of coil locations may be one or more, for example two, three, four, five, six, or more. The number may also be seven, eight, nine or more, but in some embodiments, it has been found that adding more coil locations after seven or eight locations does not necessarily translate to substantially smaller localisation volumes, such as threshold probability volumes. Measurement data corresponds to the response measurement(s) and it may include stimulation intensity threshold for excitation and/or response signals as such. Also an electromagnetic anatomical model (EAM) 120 may be provided. It may be obtained from structural imaging and, optionally, subsequent preprocessing.

The solutions may involve a specific method 130 for localising activation, in particular neuronal activation. It may be performed during and/or after the electromagnetic stimulation 110. The method may utilize, as input, any or all of the stimulation data 112, the measurement data 114 and the EAM 120. In particular, the method may utilize, as an input, minimum EFT (which may be defined as the minimal value of EFT at which activation may occur) and/or uncertainty estimate of the response signal threshold values. As an output 140, the method may provide localisation of activation. This may involve multiple concurrent activation locations so the method is not restricted to assuming that only a single location is activated at a time. Numerical and/or graphical results for activation localisation may be provided. In particular, the activation location(s) may be provided in the coordinate frame of the EAM, thereby allowing direct and accurate observation of the location(s).

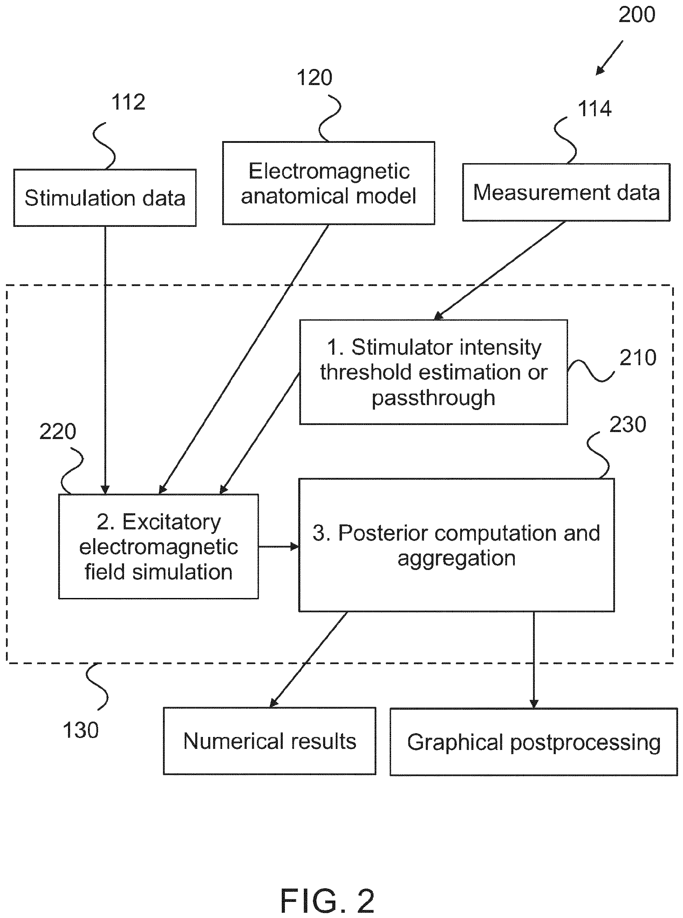

Figure 2 shows an example 200 of detailed steps involved in the METHOD part shown in Figure 1. The method may include receiving the measurement data 114, e.g. the response signals. The method may include stimulator intensity threshold estimation 210, where the received measurement data may be used. The method may also include receiving the stimulation data 112 and/or the EAM. The method may include an excitatory electromagnetic field simulation 220, where the measurement data may be used, optionally after the stimulator intensity threshold estimation. Also the stimulation data and/or the EAM may be used for the excitatory electromagnetic field simulation. From the simulation, the activation may be localised, for example in terms of one or more activation locations. This may involve posterior computation and aggregation 230.

The activation may be localised with statistics of activation parameters. The activation parameters can be used to describe the activation of the tissue excited by the stimulation. The activation parameters may comprise the activation location. As a parameter, this may then describe one or more activation locations. The activation parameters may also comprise the preferred direction of activation and/or the activating electric field threshold (EFT). The statistics of the parameters may comprise a probability distribution of the activation location. This may be also a marginal probability distribution. Importantly, the statistics of the parameters may comprise a likelihood measure that the response signals originate from a unique location-direction-EFT triple. In this case, the activation parameters may comprise or consist of all three activation parameters as described above. The statistics of the parameters may comprise a conditional expectation of the preferred direction of activation divided by the activating EFT at each (activation) location, in which case the activation parameters may again comprise or consist of all three activation parameters as described above. The statistics of the parameters may comprise a conditional expectation of activating EFT at each (activation) location, in which case the activation parameters may comprise or consist of the activation parameter and the activating EFT. The statistics of the parameters may comprise a conditional expectation of preferred direction at each (activation) location, in which case the activation parameters may comprise or consist of the activation location and the preferred direction of activation. In an embodiment, the activation parameters comprise or consist of all five activation parameters as described above. In this case, the activation parameters may comprise or consist of all three activation parameters as described above.

The response signal threshold may be inferred with any of the applicable means available to a person skilled in the art of EFT. This may involve an uncertainty, which may be utilized for localising the activation as prior information.

The activation parameters may be inferred from measured motor evoked potential (MEP) signals. The localising may be achieved by modeling the connection between activation due to an induced electric field and a measured motor threshold. The activation parameters and/or their posterior distribution may be inferred from the measurement data, for example from measured motor thresholds (MTs), for example by using Bayes' formula. The measurements may comprise or consist of active motor thresholds obtained with multiple stimulating coil locations. The posterior distribution may be sampled, for example by using a Markov chain Monte Carlo method. The plausibility of the model may be quantified, for example, by calculating the marginal likelihood of the measured thresholds. The method can be used to produce a probability distribution for the activation location, from which a minimal volume where the activation occurs with a threshold probability can be determined. The threshold probability may be predetermined. It may be 90% or higher, for example 95% or higher, or 99% or higher. The volume where this probability is exceeded, may be referred to as a threshold probability volume and it may be used as the volume to which the activation is localised. Multiple such volumes, even as separate volumes, may exist simultaneously.

For any of the solutions, in particular the methods, activation may be considered to take place if some component of the stimulating electric fields exceeds a threshold value. The method can be used to produce posterior density function(s) of the activation parameter(s) and/or statistics associated to those densities, for example given measured MT values.

An example of stimulation field model is as follows. The (brain of the) subject may be stimulated with a monophasic magnetic pulse, which will result in a primary magnetic flux density *B̅ₚ* = curl *A̅ₚ* in the brain having a reasonably fast rise time. Thus, the total electric field in the brain can be approximated by by *̅E̅*̅ = -grad*ϕ*- *∂ A̅ₚ∂t* where divσ*̅E̅*̅ = 0, and so *ϕ* is the unique function (up to a constant) in *H*¹(*D*_{brain}), *D*_{brain} being a subset of three-dimensional real-valued space, that satisfies a first equation where grad*ψ* · *σ* grad*ϕ* integrated over *x* across *D*_{brain} equals - grad*ψ* · ∂ *A̅ₚ*∂*t* integrated over x across *D*_{brain}, for all *ψ* in *H*¹(*D*_{brain}), where *σ* is the tissue conductivity and *H*¹(*D*_{brain}) is the Sobolev space consisting of square integrable functions in *D*_{brain}, with square integrable weak gradients. Notation and mathematical details are known to a person skilled in the art from text books on finite elements such as the one by Braess (Theory, fast solvers, and applications in solid mechanics, 2001).

The first equation may be solved using a multi-grid based finite element method, for example as developed in Laakso and Hirata (Fast multigrid-based computation of the induced electric field for TMS, Phys. Med. Biol., 2012), for example with 0.5 mm tri-linear cubic elements and the exciting vector potential *A̅ₚ* being that of the figure-of-eight coil, for example in Çan, Laakso, Nieminen, Murakami and Ugawa (Coil model comparison for cerebellar TMS, Biomed. Phys. Eng. Express, 2018). The conductivity data for the finite element solver may be inferred from MR images such a way that various tissues are given isotropic homogeneous conductivity values. The electric field may be interpolated at the center of each element and voxelized as an element-wise constant function.

An example of a localisation model is as follows. In particular, it can expand upon the single-site activation model of the prior art. It may be assumed that there is a location in the cerebrum (which may be interpreted as the activation location), a preferred direction (of activation), and a threshold magnitude (or activating EFT), denoted by ***r***, *̅d̅*̅ and *E*ₜₕᵣ, respectively, which are responsible for the observed MEP in a following way: The probability of a MEP being elicited at r is a monotonously increasing function w.r.t. *̅d̅*̅ · *̅E̅*̅(***r***) reaching 0.5 when *̅d̅*̅ · *E̅ (r)* = *E*ₜₕᵣ.

Now, given the activation site parameters *̅d̅*̅, r and *E*ₜₕᵣ, the MT associated with a coil configuration *k*, denoted by *tₖ*, may be defined by a second equation *tₖ* equals *E*ₜₕᵣ divided by *E̅ₖ*(***r***) *· d̅,* where *E̅ₖ* is the total induced electric field due the coil configuration *k* at the maximum stimulator intensity setting.

The rationale of such a definition is that when the stimulator intensity passes *tₖ* the MEP probability increases quickly allowing us to infer an approximate value for *tₖ* from the electromyography (EMG) measurements. As is known to a person skilled in the art, *E*ₜₕᵣ is bounded from below at the scale of 100 V m⁻¹. As an example, *E*thr > 60 V m⁻¹ = *E*_{thr,prior}, where the lower bound is deliberately chosen to impose as little prior belief as plausible. Finally, combining *E*ₜₕᵣ and *̅d̅*̅ to equation ***s*** equals *̅d̅*̅ divided by *E*ₜₕᵣ, we arrive at the model that connects activation location, (preferred) direction (for activation) and (activating electric field) threshold with minimal activation eliciting stimulation setting a third equation, where *tₖ* equals 1 divided by *E̅ₖ*(***r***) *· **s**.*

However, because *tₖ* may be inferred from noisy measurements, *tₖ, **r*** and ***s*** can be modelled as random variables, denoted by *Tₖ, R,* and *S*, respectively, and the third equation replaced with a multiplicative noise model in accordance with a fourth equation *Tₖ* equals 1 divided by *Eₖ*(*R*) *· S* times (1+*Kn*), where *n* ~ N(0, 1) and *K* > 0 is a noise parameter, typically *K* = 1/20. Such an approach may often be utilized in probabilistic methods in inverse problems.

The rationale of such a model can be based on an experimental observation that the active motor threshold (aMT) values associated with higher intensity stimulus settings tend to vary more.

Thus, the likelihood distribution of *Tₖ* given ***r*** and ***s*** is given by the equation *π*(*tₖ*|***r**, **s***) equals | *E̅ₖ*(***r***) · ***s***| times e to the power of *-***//***tₖE̅ₖ*(***r***)***s***-1**//**²/(2*K*²) divided by the square root of 2*πK²* and joint likelihood distribution of *N* coil configurations, denoting ***t*** = [*t*₁, . . . *, t_{N}*], **T** = diag(*t*₁, .. *. , t_{N}*), and **E**(***r***) = [*̅E̅*̅₁ (***r***),*̅E̅*̅₂(***r***), . . . *,E̅_{N}*(***r***)]^{T} (an element of *N*×3-dimensional real-valued space), is given by a fifth equation, in which *π*(***t***|***r,s***) equals Π*^{N}*_{*k*=1} | *E̅ₖ*(***r***) · ***s***| times e to the power of *-*//*tₖE̅ₖ*(***r***)***s***-1//²/(2*K*²) divided by the N-th power of square root of 2*πK².*

The prior distribution of *S* may be chosen according to a sixth equation, where *π_{S}*(***s***) *α q_{S}*(***s***) equals *H^{κ,ν} (*1/*E* _{thr,prior} - |***s***|), where *H*^{*κ*,ν} is a smoothed step function with parameters *κ* and v determining the approximation error to the step function. This step function may be defined by a seventh equation, where log *H*^{*κ*,ν}(*x*) equals -(log(1+e^{-2*κ*x}))^{ν}, and for numerical stability the asymptotic -(2*κx*)*^{ν}*, when 2*κx* < -10, may be used. For an example computation, values *κ* = 1000 and *v* = 4 may be used. Thus, the logprior function log *q_{S}* acts as a penalty term in the posterior log-probability density when |***s***| > 1/*E*_{thr,prior} and the prior function *q_{S}* is a very good *L*₁ estimate of the characteristic function of the set {***s*** : |***s***| < 1/*E*_{thr,prior}*}*.

The prior distribution of R may be, for example, proportional to the characteristic function of the set *D_{R}* defined in accordance of an eight equation as the intersection over *k*=1...*N* of *{**r*** as an element of *D*₀ : *αₖ*| *E̅ₖ*(***r***)| > *E*_{thr,prior}}, where *αₖ >* 1 is big enough of a stimulator intensity value that a MEP occurs certainly and *D*₀ is the intersection of the pre-central gyrus, including both grey and white matter, and a 5 cm ball centered at [-40.7,-15.7, 59.7] in Montreal Neurological Institute(MNI) coordinates. As an example, *αₖ =* 1.2*tₖ.*

Combining the likelihood, as indicated for example by the fifth equation, and priors, and in accordance to the Bayes' formula, the log-posterior density up to an additive constant is given by a ninth equation reading log *q*(***r**,**s**,**t***) equals -// **TE**(***r***)***s***-1//²/(2*K*²) + Σ*^{N}*_{*k*=1}log | *E̅ₖ*(***r***)· ***s***| - *N* log (square root of 2*πK²*) + log *π_{S}*(***s***)*.* Thus *π*(***r,s***|***t***) *α q*(***r,s,t***).

Certain *singular value coordinates* may be associated with the matrix **TE** as follows. The singular value decomposition of **TE** be written according to a tenth equation as **TE** = **Σ**³_{*i*=1} *σᵢ**u**ᵢv*^{T}*ᵢ,* where the singular values *σᵢ* > 0 may be in a descending order. Then the i:th singular value coordinate of s as an element of a three-dimensional real-valued space with respect to **TE** is given by ***s** · **v**ᵢ.* The value of such a coordinate system is that the posterior distribution *π*(***s***|***r,t***) has a special form in those coordinates. To identify the likely activation sites/locations and the preferred direction of activation, the marginal distribution *π*(***r***|***t***) proportional to the integral of *q*(***r,s,t***) over ***s*** and the conditional expectation E[***s***|***r**,**t***]*.* The latter can be approximated from the Markov chain Monte Carlo (MCMC) samples immediately. The marginal distribution calls may be obtained by computing a normalizing constant which may be calculated, for example, using path sampling, for example in accordance with Gelman and Meng (Simulating normalizing constants: from importance sampling to bridge sampling to path sampling, Stat. Sci. 1998). Path sampling allows calculating *π*(***r***|***t***) up to an unknown constant not depending on r or ***t*.**

Alternatively or additionally, the marginal probability distribution of the activation location may also be obtained, at least up to multiplicative constant, by integrating *q*(***r**,**s**,**t***) with respect to ***s*** at each candidate location for activation. In some embodiments, path sampling may still be used as it allows controlling the convergence of the computation in a stable manner: adding more MCMC samples can lead to a better approximation of the expectation and adding more integration points to the path can result in a better approximation of the relevant integral.

For localisation, a localisation threshold may be used. For this purpose, a threshold probability may be used, for example as described above. An example of a localisation statistic calculated from the posterior distribution is the *95% probability volume,* denoted here by *V*_{0.95}. However, the percentage may also be different, e.g. 90 or 99, as indicated also above. Thus, a general expression "threshold probability volume" may be used to refer to a probability volume with any threshold probability. It can be defined to be the smallest set of voxels, in terms of volume, that satisfies the equation that an integral across ***r*** over the threshold probability volume (whatever the threshold probability) of *π*(***r***|***t***) is equal or larger than the threshold probability, e.g. 0.95. Furthermore, the maximum *a-posteriori* (MAP) estimate ***r***_{MAP} of marginal distribution *π*(***r***|***t***), and/or the expected values of *E*ₜₕᵣ and ***s*** at ***r***_{MAP}, may be calculated.

The solution, and the method in particular, can thus involve a probabilistic method to localise activation, such as cortical activation, under stimulation, in particular by TMS. The activation may be localised at one or more activation locations/sites, which may be associated with response signals from the stimulation, for example MTs of TMS. The localisation may based on an elementary cortical activation model, in which an activation at a given location in the brain is considered to occur whenever electric field (even briefly) exceeds, in some preferred direction, a threshold magnitude (EFT). The (preferred) direction of activation, threshold magnitude (or activating EFT) and (activation) location are random variables whose statistics can be inferred from measurements. In particular, the MAP estimate and threshold probability volume for the activation location/site and the expectation of the preferred direction can be used. An advantage of our approach is that it provides a quantitative estimate of the plausibility of the single site activation model in terms of a marginal likelihood (MLH) value. This stands in contrast with previous approaches that have employed a similar single-site assumption. The solution allows identifying multiple different (concurrent) activation locations and/or preferred directions of activation (and/or artefacts that might cause error in the measured thresholds). The activation may be localised to any threshold probability volume, where the probability for activation is equal or larger than a threshold probability.

The localisation may be performed using a probability density function, in particular the marginal probability density function *π*(***r***|***t***), from which it is possible to evaluate if the given MAP localisation is significantly more probable than the neighboring sites or not, for example. The previous methods, instead, typically provide some specific correlation type quantity from which the localisation is inferred informally-the MAP location might coincide with other methods but it is not possible to calculate the volumes where the activation occurred with a given probability.

In the present solutions, there is no need to fix the preferred direction of activation *d̅ a priori.* Instead, it can be included as just a model parameter to be inferred from the measurements. In embodiments, it may be used as an initial assumption that the preferred direction is oriented (or close to being oriented) perpendicular to the central sulcus, thereby implying that the cortex is most sensitive when the stimulating coil is oriented perpendicular to the sulcus. The reason for existence of, and a motivation in the first place to seek, a preferred direction stems from the observation that neuronal elements in the cortex respond differently depending on the direction of the exciting electric field. As the activation site could as well be in the white matter, the directional sensitivity could be also due to the fibre orientation in it.

In addition to multiple activation locations, there may be multiple preferred directions for activation and/or activating EFTs at a single location. The present solutions allow taking this into account so that multiple preferred directions for activation and/or activating EFTs at a single location may be recognized during the activation localisation. The likelihood of the measurements can be assessed from the MLH values.

As indicated above, MT may be inferred from measurements. The threshold electric field at the activation location may be equivalent of finding a large enough MEP in the EMG signal in a second threshold probability, such as 50% or more, of the stimuli. The drawbacks of such an inference are that it requires relatively large number of stimuli to reliably detect the electric field threshold of given threshold used in the single-site activation model, and the presence of a MEP is defined in a somewhat synthetic fashion (albeit in an accordance to existing best practices): an activation site might reliably elicit a MEPs whose amplitudes were less than 100 *µ*V. However, the introduction of measurement noise in the fourth equation can be used to mitigates these problems as it is actually not necessary to accurately measure the MT.

The solutions as disclosed may be used with any physiological signal for which a threshold can be defined and measured with a known certainty. The proposed approach for finding the activation location builds upon established branch of probability theory, thus improvements of its algorithms, especially ones targeting the integration of marginal distributions, may directly translate in improvements to the proposed solutions. A consequence on building upon the probabilistic framework is that the end results of the solutions are straightforward even for non-experts to analyze.

An example of an algorithm for localising activation is as follows. Input data for the method may include any or all of the following:
- *N* electric field distributions (e.g. 3×*Mₓ* ×*M_{y}* ×*M_{z}* ×*N* array of floating point values describing total electric field due in each location to stimulation, such as TMS stimulation, at max stimulation intensity),
- *N* threshold values of readout signal (e.g. motor evoked potential - MEP), and
- a variance estimate for threshold values.
Given a function *q*(***r,s,t***) that is equivalent to the a-posteriori distribution *π*(***r,s***|***t***)*,* where ***r*** is location and ***s*** is activation sensitivity vector, the algorithm may be used to calculate any or all of the following:
1. marginal likelihood,
2. marginal distribution of activation location, integral of *π*(***r,s***|***t***) over ***s,*** for each ***r*** by integration, and
3. conditional expectation values *E_{π}*(*f*|***r**ᵢ*,***t***) for each ***r*** as integrals of *π*(***r,s***|***t***)*f*(***s***) over ***s,*** where the "moment" function f could be *f*(***s***) = ***s**, f*(***s***) = 1/|***s***|.
These may be calculated as follows:
1. enumerate the locations ***r**ᵢ: i =* 1,2,...,*Mₓ* ×*M_{y}* ×*M_{z},*
2. for each location ***r**ᵢ,* denote *g*(***s***) = *q*₁(***r**ᵢ,**s**,**t***)*,* do following in parallel:
   a. reparametrize *g*(***s***), the transformation may be denoted with *F*:
      i. the transformation *F* can be hand-tailored so that the octree (below) depth is improved to be smaller b. draw samples ***s'***₁, ***s'***₂, ... *, **s'**_{M}* from ***s'***→ *g*(*F*(***s**'*))|*det F'*(***s'***)| with No U-Turns Hamilton Markov Chain Monte Carlo method i. 100 adaptation samples and 100 actual samples.
      ii. this could also be regular Hamilton Monte Carlo method with preselected step length
3. using those samples approximate *E_{π}*(*f*|***r**ᵢ,**t***) ≈ Σ*ₖf(F(**s'**ₖ))*/*M*
4. construct an octree based on the samples (**s'**ₖ)^{N}ₖ₌₁ having following properties:
   a. each leaf should have exactly one sample unless property b. below is violated
   b. depth of the tree is at most 5
   c. the largest leaf is 0.51 times the bounding box of the samples
5. integrate the function ***s'***→ *g*(*F*(*s'*))|*det F'*(***s'***)| over the leaves of the octree using a 2nd order gaussian quadrature in each leaf. The result is a good approximation of I₁(**r**ᵢ).

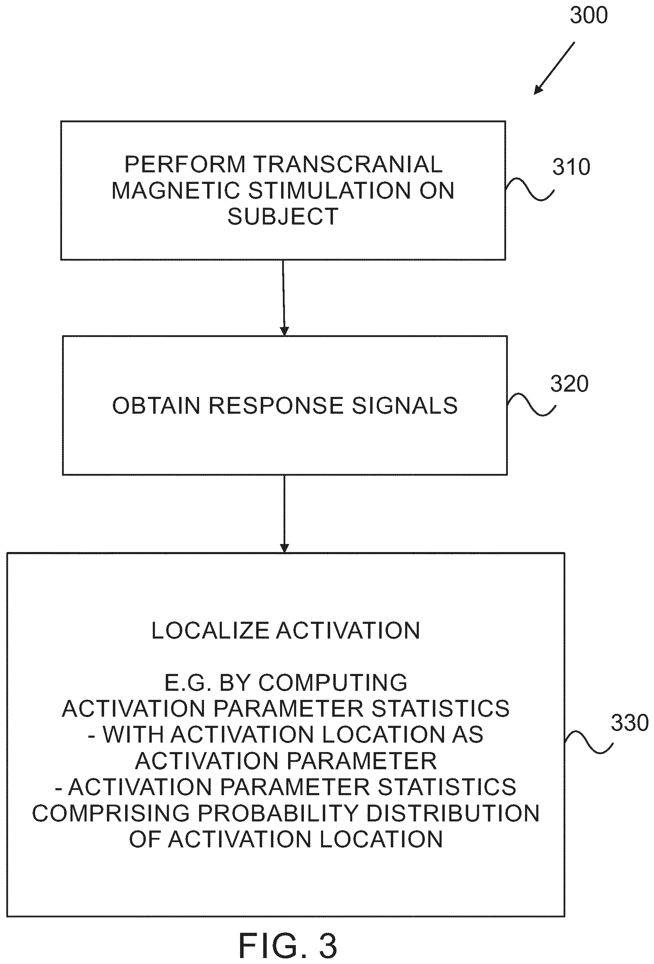

Figure 3 shows another example of a method 300. The steps of the method may be performed concurrently or subsequently. All examples disclosed above may be applied also in conjunction of the present method 300.

The method may comprise performing 310 electromagnetic stimulation, such as TMS, on a subject, in particular to a human. The method may further comprise obtaining 320 response signal(s) of the stimulation. The response signal(s) may be obtained by measurement, which may include one or more response signal threshold measurements. For the stimulation and/or obtaining the response signal(s) any method available to a person skilled in the art, in particular that of TMS, may be utilized.

While the aforementioned preparatory steps are optional, the method may comprise or consist of localising activation 330, in particular in accordance with any of the examples provided above. The method may comprise receiving the stimulation data 112 and/or measurement data 114, such as the response signal (s), and this may be subsequently used for localising the activation. The method may comprise receiving the EAM 120 and this may be subsequently used for localising and/or visualizing the activation. As previously indicated localising the activation may comprise or consist of localising cortical activation sites under the stimulation. Localising the activation may comprise computing activation parameter statistics with activation location as an activation parameter, wherein the activation parameter statistics comprises a probability distribution of the activation location. Other activation parameters and/or activation parameter statistics may also be used, for example as detailed above.

Localising the activation may be performed by a computer. For this purpose, a piece of computer software, or a computer program product, may be provided. The stimulation may be performed by any applicable apparatus available to a person skilled in the art, for example by a TMS apparatus for performing TMS. The response signal(s) may be obtained by any suitable measurement device for the stimulation. An apparatus may comprise a stimulator apparatus, such as a TMS apparatus for performing TMS, and/or a measurement device for measuring the response signal(s) from the stimulation. The apparatus, or a separate apparatus, may comprise one or more processor and one or more memories comprising computer program code. The one or more memories and the computer program code may be configured to cause the one or more processors to perform the localising of activation, for example as described herein in accordance with any of the examples.

An example of a use case of the solutions is as follows. An investigator is interested in activated cortical structures (ACS), for example corresponding to the contraction of the first dorsal interosseous (FDI) muscle of a subject. The investigator acquires conductivity segmented brain images of subject. Then the investigator pinpoints a candidate site for the ACS. Then the investigator stimulates the brain of the subject nearby ACS with a stimulator such as a TMS apparatus and records the minimal stimulator intensities values--ranging from 0% to 100%--at which activation due to stimulus, for example in FDI muscle, occurs and the stimulating coil locations and orientations. Then the investigator inputs the coil locations, orientations, and minimal stimulator intensities and conductivity map to the solution. In this use case, the coil locations and orientations can be related to "stimulation data", also illustrated in Figure 1. The conductivity map may be related to "Electromagnetic anatomical model", also illustrated in Figure 1. The minimal stimulator intensities may relate to "measurement data", also illustrated in Figure 1.

The solution can then output the statistics in graphical format where the investigator may assess the location of ACS and the EFT at given locations. The investigator may evaluate the plausibility that all stimulating coil locations and orientations stimulated a single location by assessing the likelihood value outputted by the invention.

The probability distribution of the location of the ACS may be outputted in the same coordinate system as in which the conductivity map was inputted to the solution.

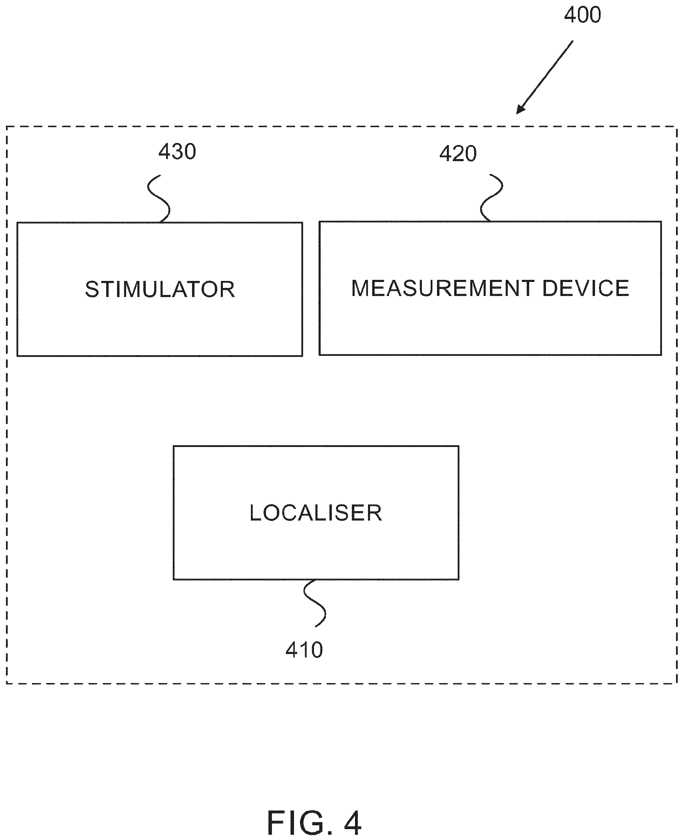

Figure 4 shows an example of an apparatus 400. The apparatus comprises, at least, the localiser 410, the device for localising the activation. This can be a computer, for example as described above. The localiser may be configured to, indirectly or directly, receive the response signal(s) from the measurement device 420 for the stimulation. It may be configured to receive additional information, for example any or all other information disclosed above for the solution, or the method in particular, e.g. the input information and/or prior information. The stimulator 430, i.e. the apparatus configured for performing the stimulation, may be a TMS apparatus for performing TMS, as described above. The stimulator and the measurement device may be separate or integrated to each other. The apparatus 400 may comprise either or both of the stimulator and the measurement device. The localiser may be integrated with either or both of the stimulator and the measurement device or it may be separate from them.

The apparatus as described above may be implemented in software, hardware, application logic or a combination of software, hardware and application logic. The application logic, software or instruction set may be maintained on any one of various conventional computer-readable media. A "computer-readable medium" may be any media or means that can contain, store, communicate, propagate or transport the instructions for use by or in connection with an instruction execution system, apparatus, or device, such as a computer. A computer-readable medium may comprise a computer-readable storage medium that may be any media or means that can contain or store the instructions for use by or in connection with an instruction execution system, apparatus, or device, such as a computer. The examples can store information relating to various processes described herein. This information can be stored in one or more memories, such as a hard disk, optical disk, magneto-optical disk, RAM, and the like. One or more databases can store the information used to implement the embodiments. The databases can be organized using data structures (e.g., records, tables, arrays, fields, graphs, trees, lists, and the like) included in one or more memories or storage devices listed herein. The databases may be located on one or more devices comprising local and/or remote devices such as servers. The processes described with respect to the embodiments can include appropriate data structures for storing data collected and/or generated by the processes of the devices and subsystems of the embodiments in one or more databases.

All or a portion of the embodiments can be implemented using one or more general purpose processors, microprocessors, digital signal processors, micro-controllers, and the like, programmed according to the teachings of the embodiments, as will be appreciated by those skilled in the computer and/or software art (s). Appropriate software can be readily prepared by programmers of ordinary skill based on the teachings of the embodiments, as will be appreciated by those skilled in the software art. In addition, the embodiments can be implemented by the preparation of application-specific integrated circuits or by interconnecting an appropriate network of conventional component circuits, as will be appreciated by those skilled in the electrical art(s). Thus, the embodiments are not limited to any specific combination of hardware and/or software.

The different functions discussed herein may be performed in a different order and/or concurrently with each other.

Any range or device value given herein may be extended or altered without losing the effect sought, unless indicated otherwise. Also any example may be combined with another example unless explicitly disallowed.

Although the subject matter has been described in language specific to structural features and/or acts, it is to be understood that the subject matter defined in the appended claims is not necessarily limited to the specific features or acts described above. Rather, the specific features and acts described above are disclosed as examples of implementing the claims and other equivalent features and acts are intended to be within the scope of the claims.

It will be understood that the benefits and advantages described above may relate to one embodiment or may relate to several embodiments. The embodiments are not limited to those that solve any or all of the stated problems or those that have any or all of the stated benefits and advantages. It will further be understood that reference to 'an' item may refer to one or more of those items.

The term 'comprising' is used herein to mean including the method, blocks or elements identified, but that such blocks or elements do not comprise an exclusive list and a method or apparatus may contain additional blocks or elements.

Numerical descriptors such as 'first', 'second', and the like are used in this text simply as a way of differentiating between parts that otherwise have similar names. The numerical descriptors are not to be construed as indicating any particular order, such as an order of preference, manufacture, or occurrence in any particular structure.

Although the invention has been described in conjunction with a certain type of apparatus and/or method, it should be understood that the invention is not limited to any certain type of apparatus and/or method. While the present inventions have been described in connection with a number of examples, embodiments and implementations, the present inventions are not so limited, but rather cover various modifications, and equivalent arrangements, which fall within the purview of the claims. Although various examples have been described above with a certain degree of particularity, or with reference to one or more individual embodiments, those skilled in the art could make numerous alterations to the disclosed examples without departing from the scope of this specification.

Notwithstanding the above, in accordance with Article 69(1) EPC, the extent of the protection conferred by a European patent or a European patent application shall be determined by the claims. Nevertheless, the description and drawings shall be used to interpret the claims.

## Claims

1. A method, executed using computing means, for localising activation under transcranial magnetic stimulation, the method comprising computing activation parameter statistics with activation location as an activation parameter, wherein the activation parameter statistics comprises a probability distribution of the activation location.

2. The method according to claim 1, wherein the activation parameter statistics are computed with preferred direction of activation as an additional activation parameter.

3. The method according to claim 1 or 2, wherein the activation parameter statistics are computed with activating electric field threshold as an additional activation parameter.

4. The method according to claim 3 when dependent on claim 2, wherein the activation parameter statistics comprises a conditional expectation of the preferred direction of activation divided by the activating electric field threshold at each activation location.

5. The method according to claim 3 or 4, wherein the activation parameter statistics comprises a conditional expectation of the activating electric field threshold at each activation location.

6. The method according to claim 2 or 4, or claim 3 or 5 when dependent on claim 2, wherein the activation parameter statistics comprises the preferred direction of activation at each activation location.

7. The method according to claim 4, claim 3 or 5 when dependent on claim 2, or claim 6 when dependent on claim 3, wherein the activation parameter statistics comprises a likelihood measure that response signals originate from a unique "activation location - preferred direction of activation - activating electric field threshold" - triple.

8. The method according to any of claims 3-5, or claim 6 or 7 when dependent on claim 3, wherein the activation parameter statistics are computed with prior information including a minimal value of electric field modulus at which activation may occur.

9. The method according to any preceding claim, wherein the activation parameter statistics are computed with prior information including accuracy information about response signals.

10. The method according to any preceding claim, wherein the activation is localised within one or more threshold probability volumes for which an integral of the probability distribution exceeds a threshold probability.

11. The method according to claim 10, wherein the threshold probability is 90 percent or more.

12. A computer program product comprising instructions which, when the program is executed by a computer, cause the computer to carry out the method of any preceding claim.

## Patentansprüche

1. Verfahren, das unter Verwendung von Rechenmitteln ausgeführt wird, zur Lokalisierung der Aktivierung unter transkranieller Magnetstimulation, das Verfahren umfassend das Berechnen einer Aktivierungsparameterstatistik mit dem Aktivierungsort als Aktivierungsparameter, wobei die Aktivierungsparameterstatistik eine Wahrscheinlichkeitsverteilung des Aktivierungsorts umfasst.

2. Verfahren nach Anspruch 1, wobei die Aktivierungsparameterstatistik mit einer bevorzugten Aktivierungsrichtung als zusätzlichem Aktivierungsparameter berechnet wird.

3. Verfahren nach Anspruch 1 oder 2, wobei die Aktivierungsparameterstatistik mit einer aktivierenden elektrischen Feldschwelle als zusätzlichem Aktivierungsparameter berechnet wird.

4. Verfahren nach Anspruch 3, wenn abhängig von Anspruch 2, wobei die Aktivierungsparameterstatistik eine bedingte Erwartung der bevorzugten Aktivierungsrichtung geteilt durch die aktivierende elektrische Feldschwelle an jedem Aktivierungsort umfasst.

5. Verfahren nach Anspruch 3 oder 4, wobei die Aktivierungsparameterstatistik eine bedingte Erwartung der aktivierenden elektrischen Feldschwelle an jedem Aktivierungsort umfasst.

6. Verfahren nach Anspruch 2 oder 4 oder Anspruch 3 oder 5, wenn abhängig von Anspruch 2, wobei die Aktivierungsparameterstatistik die bevorzugte Aktivierungsrichtung an jedem Aktivierungsort umfasst.

7. Verfahren nach Anspruch 4, Anspruch 3 oder 5, sofern abhängig von Anspruch 2, oder Anspruch 6, sofern abhängig von Anspruch 3, wobei die Aktivierungsparameterstatistik ein Wahrscheinlichkeitsmaß dafür umfasst, dass Antwortsignale von einem eindeutigen Tripel "Aktivierungsort - bevorzugte Aktivierungsrichtung - aktivierende elektrische Feldschwelle" stammen.

8. Verfahren nach einem der Ansprüche 3 bis 5 oder Anspruch 6 oder 7, wenn abhängig von Anspruch 3, wobei die Aktivierungsparameterstatistik mit Vorabinformationen berechnet wird, die einen Mindestwert eines elektrischen Feldmoduls enthalten, bei dem eine Aktivierung erfolgen kann.

9. Verfahren nach einem der vorhergehenden Ansprüche, wobei die Aktivierungsparameterstatistik mit Vorabinformationen berechnet wird, die Genauigkeitsinformationen zu Antwortsignalen enthalten.

10. Verfahren nach einem der vorhergehenden Ansprüche, wobei die Aktivierung innerhalb eines oder mehrerer Schwellenwahrscheinlichkeitsvolumina lokalisiert ist, für die ein Integral der Wahrscheinlichkeitsverteilung eine Schwellenwahrscheinlichkeit überschreitet.

11. Verfahren nach Anspruch 10, wobei die Schwellenwahrscheinlichkeit 90 Prozent oder mehr beträgt.

12. Computerprogrammprodukt mit Anweisungen, welche bei Ausführung durch einen Computer den Computer veranlassen, das Verfahren nach einem der vorhergehenden Ansprüche auszuführen.

## Revendications

1. Procédé, exécuté à l'aide de moyens informatiques, pour localiser une activation sous stimulation magnétique transcrânienne, TMS, le procédé comprenant une réception de données de réponse provenant de la TMS et, en se basant sur lesdites données, un calcul de statistiques de paramètre d'activation avec un emplacement d'activation en tant que paramètre d'activation, dans lequel les statistiques de paramètre d'activation comprennent une distribution de probabilités de l'emplacement d'activation.

2. Procédé selon la revendication 1, dans lequel les statistiques de paramètre d'activation sont calculées avec une direction préférée d'activation en tant que paramètre d'activation additionnel.

3. Procédé selon la revendication 1 ou 2, dans lequel les statistiques de paramètre d'activation sont calculées avec un seuil de champ électrique d'activation, EFT, en tant que paramètre d'activation additionnel.

4. Procédé selon la revendication 3 lorsqu'elle dépend de la revendication 2, dans lequel les statistiques de paramètre d'activation comprennent une attente conditionnelle de la direction préférée d'activation divisée par l'EFT d'activation au niveau de chaque emplacement d'activation.

5. Procédé selon la revendication 3 ou 4, dans lequel les statistiques de paramètre d'activation comprennent une attente conditionnelle de l'EFT d'activation au niveau de chaque emplacement d'activation.

6. Procédé selon la revendication 2 ou 4, ou la revendication 3 ou 5 lorsqu'elle dépend de la revendication 2, dans lequel les statistiques de paramètre d'activation comprennent la direction préférée d'activation au niveau de chaque emplacement d'activation.

7. Procédé selon la revendication 4, la revendication 3 ou 5 lorsqu'elle dépend de la revendication 2, ou la revendication 6 lorsqu'elle dépend de la revendication 3, dans lequel les statistiques de paramètre d'activation comprennent une mesure de probabilité que des signaux de réponse proviennent d'un triplet unique "emplacement d'activation - direction préférée d'activation - EFT d'activation".

8. Procédé selon l'une quelconque des revendications 3 à 5, ou la revendication 6 ou 7 lorsqu'elle dépend de la revendication 3, dans lequel les statistiques de paramètre d'activation sont calculées avec des informations préalables incluant une valeur minimale de module de champ électrique au niveau de laquelle une activation peut se produire.

9. Procédé selon une quelconque revendication précédente, dans lequel les statistiques de paramètre d'activation sont calculées avec des informations préalables incluant des informations relatives à la précision concernant des signaux de réponse.

10. Procédé selon une quelconque revendication précédente, dans lequel l'activation est localisée au sein d'un ou plusieurs volumes de probabilités de seuil pour lesquels une intégrale de la distribution de probabilités dépasse une probabilité de seuil.

11. Procédé selon la revendication 10, dans lequel la probabilité de seuil est de 90 pour cent ou plus.

12. Produit programme informatique comprenant des instructions qui, lorsque le programme est exécuté par un ordinateur, amènent l'ordinateur à effectuer le procédé selon une quelconque revendication précédente.
